(19) Europäisches Patentamt / European Patent Office / Office européen des brevets

(11) **EP 3 395 824 A1**

(12) **EUROPEAN PATENT APPLICATION**
published in accordance with Art. 153(4) EPC

(43) Date of publication:
**31.10.2018 Bulletin 2018/44**

(51) Int Cl.:
*C07H 3/06* (2006.01)     *B09B 3/00* (2006.01)
*C08B 15/02* (2006.01)     *C13B 10/00* (2011.01)
*C13K 13/00* (2006.01)     *C07B 61/00* (2006.01)

(21) Application number: **16875664.1**

(22) Date of filing: **14.12.2016**

(86) International application number:
**PCT/JP2016/087161**

(87) International publication number:
**WO 2017/104687 (22.06.2017 Gazette 2017/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**MA MD**

(30) Priority: **18.12.2015 JP 2015247401**

(71) Applicants:
• **Showa Denko K.K.**
  **Tokyo 105-8518 (JP)**
• **National University Corporation**
  **Hokkaido University**
  **Sapporo-shi, Hokkaido 060-0808 (JP)**

(72) Inventors:
• **FUJITA Ichiro**
  **Tokyo 105-8518 (JP)**
• **FUKUOKA Atsushi**
  **Sapporo-shi**
  **Hokkaido 060-0808 (JP)**
• **KOBAYASHI Hirokazu**
  **Sapporo-shi**
  **Hokkaido 060-0808 (JP)**
• **SHROTRI Abhijit**
  **Sapporo-shi**
  **Hokkaido 060-0808 (JP)**

(74) Representative: **Strehl Schübel-Hopf & Partner**
  **Maximilianstrasse 54**
  **80538 München (DE)**

(54) **METHOD FOR MANUFACTURING CELLOOLIGOSACCHARIDE**

(57)     The present invention provides a method of manufacturing a cellooligosaccharide containing an oligomer having a degree of polymerization of from 3 to 6, the method comprising performing a reaction involving heating and hydrolyzing plant biomass in coexistence with a carbon catalyst and water under such a condition that a temperature-time product over a range of from 170°C to 230°C in a graph for showing a relationship between a reaction temperature on an axis of ordinate and a reaction time on an axis of abscissa is from 80°C·min to 365°C·min. The maximum reaction temperature (X) and the retention time (Y) at the maximum reaction temperature preferably satisfy a relationship represented by the following expressions (1) to (3).

$$Y > -0.0714X + 15 \qquad (1)$$

$$Y < -X + 210 \qquad (2)$$

$$X > 175 \qquad (3)$$

## EP 3 395 824 A1

**Description**

Technical Field

The present invention relates to a method of manufacturing a cellooligosaccharide containing an oligomer having a degree of polymerization of glucose of from 3 to 6 through a hydrolysis reaction of plant biomass using a carbon catalyst.

Background Art

A cellooligosaccharide is an oligosaccharide obtained through polymerization of glucose by β1,4 bond. In recent years, the cellooligosaccharide has been found to have functionalities, such as moisture retention, stickiness suppression, refreshing taste impartment, starch retrogradation reduction, and protein denaturation suppression, and has been expected to be utilized in the fields of medicines, cosmetics, food, and feed.

In particular, higher expectations have been placed on a cellooligosaccharide having a degree of polymerization of glucose of 3 or more, in enhancing the above-mentioned functionalities and imparting novel functionalities.

However, a currently industrially utilized cellooligosaccharide, which is manufactured through an enzymatic reaction, hardly contains cellotriose, which is a trimer, or a higher oligomer, with its main components being glucose and cellobiose, which is a dimer (JP 2009-189293 A; Patent Literature 1).

As a cellooligosaccharide-manufacturing technology other than the enzymatic method, there are known a hydrothermal treatment method (JP 2011-068578 A (US 2012/232264 A1); Patent Literature 2, WO 2011/036955 A1 (US 9144785 B2); Patent Literature 3, WO 2012/128055 A1 (US 9284614 B2); Patent Literature 4), and a hydrothermal treatment method using oxidizing water containing hypochlorous acid (JP 2006-320261 A; Patent Literature 5). However, in each of the patent literatures, the cellooligosaccharide is treated as an intermediate product in a process of degrading cellulose into glucose, and there is no disclosure of specific data on its yield or the like. That is, there is no established method of industrially manufacturing an oligomer having a degree of polymerization of glucose of from 3 to 6 with good efficiency. Hitherto, the oligomer has only been manufactured by purifying the target product contained in a slight amount in, for example, a hydrolysis reaction liquid containing glucose as a main component.

Under the above-mentioned circumstances, there is a demand for establishment of a manufacturing method by which a cellooligosaccharide containing the oligomer having a degree of polymerization of glucose of from 3 (cellotriose) to 6, which is not industrially produced at present, is obtained in high yield.

Citation List

Patent Literature

[PTL 1] JP 2009-189293 A
[PTL 2] JP 2011-068578 A (US 2012/232264 A1)
[PTL 3] WO 2011/036955 A1 (US 9144785 B2)
[PTL 4] WO 2012/128055 A1 (US 9284614 B2)
[PTL 5] JP 2006-320261 A

Summary of Invention

Technical Problem

It is an object of the present invention to provide a method of manufacturing a cellooligosaccharide containing an oligomer having a degree of polymerization of glucose of from 3 to 6 in a method of hydrolyzing plant biomass using a carbon catalyst.

Solution to Problem

The inventors of the present invention have found that a cellooligosaccharide containing an oligomer having a degree of polymerization of glucose of from 3 to 6 can be manufactured by performing, in a hydrolysis reaction of plant biomass using a carbon catalyst, a hydrothermal reaction through the control of a temperature increase rate, a cooling rate, a reaction temperature, and a reaction time. Thus, the inventors have completed the present invention.

That is, the present invention relates to a method of manufacturing a cellooligosaccharide according to the following items [1] to [9].

[1] A method of manufacturing a cellooligosaccharide containing an oligomer having a degree of polymerization of from 3 to 6, the method comprising performing a reaction involving heating and hydrolyzing plant biomass in coexistence with a carbon catalyst and water under such a condition that a temperature-time product over a range of from 170°C to 230°C in a graph for showing a relationship between a reaction temperature on an axis of ordinate and a reaction time on an axis of abscissa is from 80°C·min to 365°C·min.

[2] The method of manufacturing a cellooligosaccharide according to [1] above, in which a maximum reaction temperature (X) and a retention time (Y) at the maximum reaction temperature satisfy a relationship represented by the following expressions (1) to (3):

$$Y > -0.0714X + 15 \qquad (1)$$

$$Y < -X + 210 \qquad (2)$$

$$X > 175 \qquad (3)$$

where X represents the maximum reaction temperature (°C), and Y represents the retention time (min) at the maximum reaction temperature.

[3] The method of manufacturing a cellooligosaccharide according to [1] or [2] above, in which a temperature increase rate during heating to the maximum reaction temperature, and a cooling rate during cooling from the maximum reaction temperature are each from 0.1°C/min to 30°C/min.

[4] The method of manufacturing a cellooligosaccharide according to any one of [1] to [3] above, in which the carbon catalyst is activated carbon.

[5] The method of manufacturing a cellooligosaccharide according to any one of [1] to [4] above, in which the carbon catalyst is steam-activated carbon.

[6] The method of manufacturing a cellooligosaccharide according to any one of [1] to [4] above, in which the carbon catalyst is air-oxidized steam-activated carbon and/or air-oxidized chemical-activated carbon.

[7] The method of manufacturing a cellooligosaccharide according to any one of [1] to [6] above, in which the plant biomass and the carbon catalyst are mixed and pulverized in advance before being used as a raw material.

[8] The method of manufacturing a cellooligosaccharide according to any one of [1] to [7] above, in which the plant biomass is pulverized in advance before being used as a raw material.

[9] The method of manufacturing a cellooligosaccharide according to any one of [1] to [8] above, in which the reaction is terminated at a time point when a yield of an oligosaccharide having a degree of polymerization of glucose of from 3 to 6 is from 50% to 80%.

Advantageous Effects of Invention

According to the present invention, the cellooligosaccharide containing an oligomer having a degree of polymerization of glucose of from 3 to 6 can be manufactured from plant biomass using a carbon catalyst.

Brief Description of Drawings

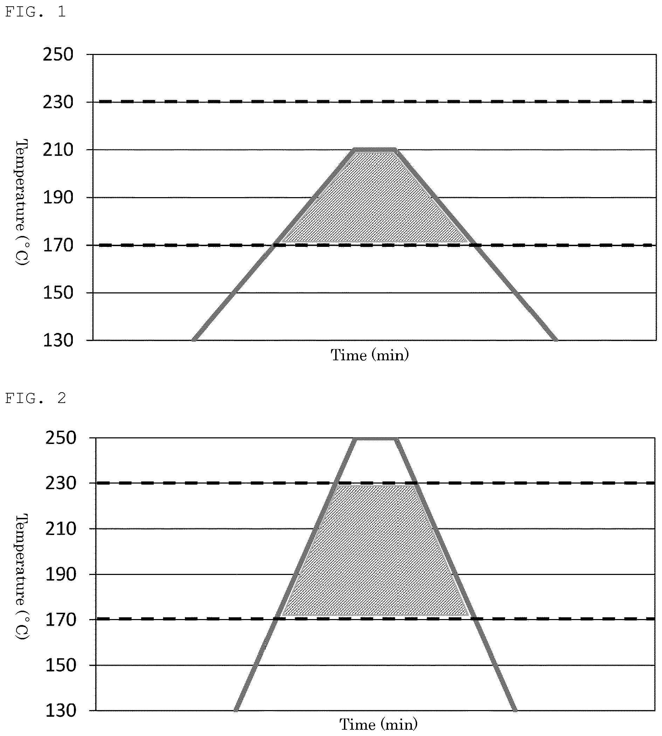

FIG. 1 is a graph for schematically showing the temperature transition of a hydrolysis reaction in which the maximum reaction temperature is less than 230°C, in which the axis of abscissa represents elapsed time (min), the axis of ordinate represents temperature (°C), and a temperature-time product (°C·min) over the range of from 170°C to 230°C is shown as a shaded area.

FIG. 2 is a graph for schematically showing the temperature transition of a hydrolysis reaction in which the maximum reaction temperature is more than 230°C, in which the axis of abscissa represents elapsed time (min), the axis of ordinate represents temperature (°C), and a temperature-time product (°C·min) over the range of from 170°C to 230°C is shown as a shaded area.

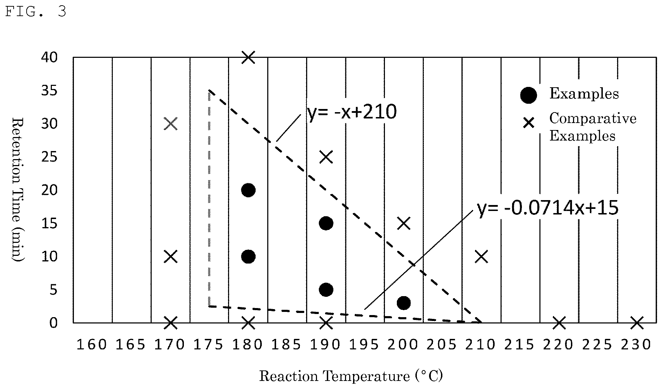

FIG. 3 is a graph for showing results obtained by a method of the present invention carried out at various reaction temperatures with steam-activated carbons (BA50 and SD50) and chemical-activated carbon (Zn60) (results of Examples 1 to 7 and Comparative Examples 1 to 12).

Description of Embodiments

A method according to a preferred embodiment of the present invention is described below. The embodiment to be described below is a typical example of the present invention, the present invention is not limited thereto, and the embodiment to be described below should not be construed as limiting the scope of the present invention.

[Plant Biomass (Solid Substrate)]

The term "biomass" generally refers to "recyclable organic resource of biologic origin, excluding fossil resources", but the "plant biomass" (hereinafter sometimes referred to as solid substrate) to be used in the present invention is, for example, biomass mainly containing cellulose or hemicellulose, such as rice straw, straw, sugarcane straw, chaff, bagasse, a broadleaf tree, bamboo, a coniferous tree, kenaf, furniture waste, construction waste, waste paper, or a food residue.

The plant biomass to be used may be plant biomass subjected to purification treatment or plant biomass not subjected to purification treatment. The plant biomass subjected to purification treatment is, for example, one that is obtained by subjecting the plant biomass to treatment such as alkali steam treatment, alkaline sulfite steam treatment, neutral sulfite steam treatment, alkaline sodium sulfide steam treatment, or ammonia steam treatment, and then to delignification treatment by solid-liquid separation and water washing, and that contains cellulose. Further, the plant biomass may be industrially prepared cellulose or the like.

The plant biomass may contain an ash content such as silicon, aluminum, calcium, magnesium, potassium, or sodium, which is derived from the raw material, as an impurity.

The plant biomass may be in a dry form or a wet form, and may be crystalline or non-crystalline. It is desired that the plant biomass be pulverized prior to a reaction. The pulverization increases a contact property with a solid catalyst, and thus promotes a hydrolysis reaction. Therefore, it is desired that the plant biomass have a shape and size appropriate for the pulverization. As such shape and size, there is given, for example, a powder shape having a particle diameter of from 20 $\mu$m to 1,000 $\mu$m.

[Solid Catalyst]

The solid catalyst is not particularly limited as long as the catalyst can catalyze the hydrolysis of the plant biomass, but is preferably a carbon material having activity to hydrolyze glycoside bonds typified by $\beta$-1,4 glycosidic bonds between glucose units that form cellulose contained as a main component.

Examples of the carbon material include activated carbon, carbon black, graphite, and air-oxidized wood powder. Those carbon materials may be used alone or in combination thereof. Regarding the shape of the carbon material, from the viewpoint of improving reactivity by increasing an area for contact with a substrate, the carbon material is preferably porous and/or particulate. From the viewpoint of promoting hydrolysis by expressing an acid center, a carbon material having a functional group, such as a phenolic hydroxyl group, a carboxyl group, a sulfo group, or a phosphate group, in its surface is preferred.

As a porous carbon material having a functional group in its surface, there are given: a wood material, such as coconut husk, eucalyptus, bamboo, pine, walnut husk, or bagasse; and activated carbon prepared by a method involving treating coke or phenol at high temperature with a gas, such as steam, carbon dioxide, or air (physical method), or by a method involving treating coke or phenol at high temperature with a chemical, such as an alkali or zinc chloride (chemical method). The examples further include carbon materials each obtained by uniformly subjecting a wood material or activated carbon to heat treatment in the presence of air (to air oxidization).

From the viewpoint that the yield of an oligosaccharide having a degree of polymerization equal to or higher than that of cellotriose is high, it is preferred that steam-activated carbon, chemical-activated carbon, air-oxidized wood powder, air-oxidized steam-activated carbon, or air-oxidized chemical-activated carbon be used.

[Pulverization of Plant Biomass]

Cellulose, which is a main component of plant biomass, exhibits crystallinity, because two or more cellulose molecules are bonded to each other through hydrogen bonding. In the present invention, such cellulose exhibiting crystallinity may be used as it is as a raw material, but cellulose that is subjected to treatment for reducing crystallinity and thus has reduced crystallinity is preferably used. As the cellulose having reduced crystallinity, cellulose in which the crystallinity is partially reduced or cellulose in which the crystallinity is completely or almost completely lost may be used. The kind of the treatment for reducing crystallinity is not particularly limited, but treatment for reducing crystallinity capable of breaking the hydrogen bonding to at least partially generate a single-chain cellulose molecule is preferred. By using as the raw material cellulose at least partially containing the single-chain cellulose molecule, hydrolysis efficiency can be significantly improved.

As a method of physically breaking the hydrogen bonding between cellulose molecules, there is given, for example, pulverization treatment. Pulverization means is not particularly limited as long as the means has a function to enable fine pulverization. For example, the mode of a pulverization apparatus may be a dry mode or a wet mode. In addition, the pulverization system of the apparatus may be a batch system or a continuous system. Further, the pulverization force of the apparatus may be provided by any of impact, compression, shearing, friction, and the like.

Examples of the apparatus to be used in the pulverization treatment include: tumbling ball mills, such as a pot mill, a tube mill, and a conical mill; vibrating ball mills, such as a circular vibration type vibration mill, a rotary vibration mill, and a centrifugal mill; mixing mills, such as a media agitating mill, an annular mill, a circulation type mill, and a tower mill; jet mills, such as a spiral flow jet mill, an impact type jet mill, a fluidized bed type jet mill, and a wet type jet mill; shear mills, such as a Raikai mixer and an angmill; colloid mills, such as a mortar and a stone mill; impact mills, such as a hammer mill, a cage mill, a pin mill, a disintegrator, a screen mill, a turbo mill, and a centrifugal classification mill; and a planetary ball mill, which is a mill of a type that employs rotation and revolution movements.

A hydrolysis reaction of plant biomass using a carbon catalyst is a reaction between a solid substrate and a solid catalyst, and a rate of the reaction is limited by contact between the substrate and the catalyst. Therefore, an effective method of improving reactivity involves preliminarily mixing the solid substrate and the solid catalyst and then performing simultaneous pulverization treatment.

The simultaneous pulverization treatment may include pre-treatment for reducing the crystallinity of the substrate in addition to the mixing. From such viewpoint, the pulverization apparatus to be used is preferably a tumbling ball mill, a vibrating ball mill, a mixing mill, or a planetary ball mill, which is used for the pre-treatment for reducing the crystallinity of the substrate, more preferably a pot mill classified as the tumbling ball mill, a media agitating mill classified as the mixing mill, or the planetary ball mill. Further, a raw material having a higher bulk density obtained by the simultaneous pulverization treatment for the solid catalyst and the solid substrate tends to have higher reactivity. Therefore, it is more preferred to use the tumbling ball mill, the mixing mill, or the planetary ball mill that can apply a strong compression force enough to allow a pulverized product of the solid catalyst to dig into a pulverized product of the solid substrate.

In each of the raw material obtained by separately pulverizing the solid substrate and the raw material obtained by simultaneously pulverizing the substrate and the catalyst, the average particle diameter after the fine pulverization (median diameter: particle diameter (D50) at a point where the cumulative volume curve determined based on the total powder volume defined as 100% crosses 50%) is from 1 $\mu$m to 100 $\mu$m, and is preferably from 1 $\mu$m to 30 $\mu$m, more preferably from 1 $\mu$m to 20 $\mu$m from the viewpoint of improving reactivity.

When the particle diameter of a raw material to be subjected to fine pulverization treatment is large, in order to efficiently perform the fine pulverization, preliminary pulverization treatment is preferably performed before the fine pulverization. The preliminary pulverization treatment may be performed with, for example: a coarse crusher, such as a shredder, a jaw crusher, a gyratory crusher, a cone crusher, a hammer crusher, a roll crusher, or a roll mill; or a medium crusher, such as a stamp mill, an edge runner, a cutting/shearing mill, a rod mill, an autogenous mill, or a roller mill. The time for treating the raw material is not particularly limited as long as the raw material can be homogeneously and finely pulverized by the treatment.

A ratio between the solid catalyst and the solid substrate is not particularly limited in any of the case where the substrate is separately pulverized and the case where the substrate and the catalyst are simultaneously pulverized. However, from the viewpoints of the hydrolysis efficiency in the reaction, the decrease of a substrate residue after the reaction, and the recovery rate of the produced saccharide, the ratio of the solid catalyst is preferably from 1 part by mass to 100 parts by mass, more preferably from 1 part by mass to 10 parts by mass with respect to 100 parts by mass of the solid substrate.

[Hydrolysis Reaction]

The hydrolysis reaction using plant biomass as a substrate to produce the cellooligosaccharide containing an oligomer having a degree of polymerization of glucose of from 3 to 6 is performed by heating the substrate in the presence of a catalyst and water, preferably at a temperature at which a pressurized state is achieved.

The heating degrades cellulose into glucose, which is a monosaccharide, via the cellooligosaccharide, and when the reaction further proceeds, an excessive degradation product, such as 5-hydroxymethylfurfural, is produced. Therefore, in order to achieve the object of the present invention, the hydrolysis reaction needs to be performed under such a condition that cellulose is degraded into the cellooligosaccharide, but thereafter, the cellooligosaccharide is not degraded into glucose.

The conditions of the hydrolysis reaction for achieving the object of the present invention are as described below.

The hydrolysis reaction may be performed at a temperature of from 170°C to 230°C. The maximum temperature may exceed 230°C temporarily as long as the effect of the present invention is not impaired.

When the area in the range of from 170°C to 230°C in a graph in which the axis of ordinate represents reaction temperature and the axis of abscissa represents time (see FIG. 1 and FIG. 2) (hereinafter referred to as "temperature-time product over 170°C to 230°C") is from 80°C·min to 365°C·min, the cellooligosaccharide having a degree of polymerization of glucose of from 3 to 6 can be efficiently obtained. In particular, the temperature-time product over 170°C to 230°C is preferably from 80°C·min to 300°C·min, more preferably from 80°C·min to 250°C·min.

The temperature-time product over 170°C to 230°C may be determined by integrating the reaction temperature with respect to time over the range of from 170°C to 230°C. In the case of a reaction pattern in which the temperature increase rate during heating and the cooling rate during cooling are constant, the temperature-time product may be determined with the following equation through trapezoidal approximation.

```
Temperature-time product over 170°C to 230°C = {(maximum
reaction temperature - 170°C) ÷ temperature increase rate
(°C/min) × (maximum reaction temperature - 170°C) ÷
2)+{(maximum reaction temperature - 170°C) × retention time
(min) at maximum reaction temperature}+{(maximum reaction
temperature - 170°C) ÷ cooling rate (°C/min) × (maximum
reaction temperature - 170°C)÷2)}
```

When the maximum reaction temperature is more than 230°C, the calculation is performed on the assumption that the "maximum reaction temperature" in the equation is 230°C (see FIG. 2).

A case in which the temperature-time product over 170°C to 230°C is less than 80°C·min is not preferred because degradation of cellulose into the cellooligosaccharide does not proceed. In addition, a case in which the temperature-time product over 170°C to 230°C is more than 365°C·min is not preferred because degradation of the cellooligosaccharide into glucose and excessive degradation of glucose into 5-hydroxymethylfurfural or the like rapidly proceed.

Further, the hydrolysis reaction is preferably performed under such a condition that the maximum reaction temperature (X) and the retention time (Y) at the maximum reaction temperature satisfy a relationship represented by the following expressions (1) to (3).

$$Y > -0.0714X + 15 \qquad (1)$$

$$Y < -X + 210 \qquad (2)$$

$$X > 175 \qquad (3)$$

In the expressions, X represents the maximum reaction temperature (°C), and Y represents the retention time (min) at the maximum reaction temperature.

A case in which the maximum reaction temperature (X) of the hydrolysis and the retention time (Y) at the maximum reaction temperature fall within the above-mentioned range is preferred because degradation of cellulose into the cellooligosaccharide proceeds, and at the same time, degradation of the cellooligosaccharide into glucose and excessive degradation of glucose into 5-hydroxymethylfurfural or the like are suppressed.

In particular, the maximum reaction temperature of the hydrolysis falls within the range of preferably from 180°C to 200°C, more preferably from 180°C to 190°C.

The temperature increase rate during heating to the reaction temperature, and the temperature decrease rate during cooling after the termination of the heating at the reaction temperature may each be, for example, a rate in the range of from 0.1°C/min to 30°C/min, more suitably in the range of preferably from 0.2°C/min to 20°C/min. A rate of less than 0.1°C/min is not preferred because the hydrolysis reaction proceeds excessively during the temperature increase process or the cooling process, and a rate of more than 30°C/min is not preferred because equipment cost increases owing to enhancement of equipment capacity.

The amount of water present during the hydrolysis is an amount that allows at least the hydrolysis of the total amount of cellulose. In consideration of, for example, the fluidity and stirring property of the reaction mixture, the amount may be set to the range of from 1 to 500, preferably the range of from 2 to 200 in terms of mass ratio to the cellulose.

The hydrolysis is influenced by pH. In the present invention, the hydrolysis reaction is preferably performed under the condition of a pH of from 4 to 7. When the pH is less than 4, degradation of the cellooligosaccharide into glucose is liable to proceed, and when the pH is more than 7, the hydrolysis is inhibited. Therefore, in order to increase the yield of the cellooligosaccharide, the pH at the start of the reaction is appropriately adjusted to the above-mentioned range.

The atmosphere of the hydrolysis is not particularly limited. From an industrial viewpoint, the hydrolysis is preferably carried out under an air atmosphere, or may be carried out under an atmosphere of gas other than air, such as oxygen, nitrogen, or hydrogen, or a mixture thereof.

In the method of the present invention, the hydrolysis of the cellulose may be carried out in a batch fashion or a continuous fashion.

A batch reaction is performed while stirring a reaction mixture in a sealed vessel, such as an autoclave. In this case, because of the sealed state, even when the reaction is started at normal pressure, the reaction system is brought into a pressurized state when heated at the above-mentioned temperature. Further, the reaction may be performed under a state in which the inside of the sealed vessel is pressurized before the reaction or during the reaction. The inside of the sealed vessel is pressurized to a pressure of, for example, from 0.1 MPa to 30 MPa, preferably from 1 MPa to 20 MPa, more preferably from 2 MPa to 10 MPa.

The hydrolysis reaction is preferably terminated at a time point when the yield of the oligosaccharide having a degree of polymerization of glucose of from 3 (cellotriose) to 6 is between 10% to 80%. The time point varies depending on, for example, the heating temperature, the kind and amount of the catalyst to be used, the amount of water (ratio to cellulose), the kind of the cellulose, and a stirring method and conditions thereof. However, those conditions can be determined through a preliminary experiment.

Examples

(1) Solid Catalyst and Solid Substrate

In the following Examples and Comparative Examples, steam-activated carbon BA50 (manufactured by Ajinomoto Fine-Techno Co., Inc.) or SD50 (manufactured by Ajinomoto Fine-Techno Co., Inc.), or chemical-activated carbon ZN60 (manufactured by Ajinomoto Fine-Techno Co., Inc.) activated with zinc chloride, which served as a solid catalyst, and Avicel (microcrystalline cellulose manufactured by Merck), which served as a solid substrate, were each directly used, or were subjected to pulverization treatment by a method described in the following (2) before use.

(2) Mixed and Pulverized Raw Material

10.00 g of Avicel (microcrystalline cellulose manufactured by Merck), which served as the solid substrate, and 1.54 g of the solid catalyst (mass ratio between the substrate and the catalyst: 6.5:1.0) were loaded in a 3,600 mL-volume ceramic pot mill together with 2,000 g of alumina balls each having a diameter of 1.5 cm. The ceramic pot mill was set to a desktop pot mill rotating table (manufactured by Nitto Kagaku Co., Ltd., desktop pot mill type ANZ-51S). The substrate and the catalyst were mixed and simultaneously pulverized through ball mill treatment at 60 rpm for 48 hours. The obtained raw material is hereinafter abbreviated as mixed and pulverized raw material.

Examples 1 to 7 and Comparative Examples 1 to 12: Hydrolysis Reaction

Catalysts and a substrate shown in Table 1 were used. 0.374 g of a mixed and pulverized raw material (2.00 mmol in terms of $C_6H_{10}O_5$) and 40 mL of water, or 0.324 g of an unpulverized substrate, 0.050 g of a catalyst, and 40 mL of water were put in a high-pressure reactor (internal volume: 100 mL, autoclave manufactured by OM Lab-tech Co., Ltd, made of Hastelloy C22), and then heated from room temperature to a reaction temperature shown in Table 1 at from 10°C/min to 30°C/min (average temperature increase rate: 11.3°C/min) while being stirred at 600 rpm. After the maximum reaction temperature was reached, the aqueous dispersion was retained at the temperature for a time period shown in Table 1. After that, the heating was stopped and the reactor was air-cooled at from 10°C/min to 30°C/min (average temperature decrease rate: 16.7°C/min). After the cooling, the reaction liquid was separated with a centrifuge into a liquid and a solid, and the supernatant sample was analyzed. A reaction time of "0 minutes" means that cooling is started the moment the maximum reaction temperature is reached.

[Quantification of Product]

As products in a liquid-phase sample from which a solid had been removed, glucose and oligosaccharides from cellobiose to cellohexaose were quantitatively analyzed with a high-performance liquid chromatograph manufactured by Shimadzu Corporation (conditions 1: column: Shodex (trademark) SH-1011, mobile phase: water at 0.5 mL/min, 50°C, detection: differential refractive index).
An equation for calculating a yield is shown below.

```
Product yield (%) = {(amount of substance of carbon in
component of interest)/(amount of substance of carbon in added
cellulose)}×100
```

[Analysis Results]

The results are shown in Table 1.

Table 1

| | Reaction conditions | | | | | | Results | | |
|---|---|---|---|---|---|---|---|---|---|
| | Catalyst | Substrate | Pulverization | Temperature (°C) | Time (min) | Temperature -time product (°C·min) | Yield of G3 to G6 (%) | Yield of G2 (%) | Yield of glucose (%) |
| Comparative Example 1 | BA50 | Avicel | Mixing | 230 | 0.0 | 534 | 0 | 5 | 51 |
| Comparative Example 2 | BA50 | Avicel | Mixing | 220 | 0.0 | 371 | 16 | 19 | 37 |
| Comparative Example 3 | BA50 | Avicel | Mixing | 210 | 10.0 | 637 | 0 | 2 | 21 |
| Comparative Example 4 | BA50 | Avicel | Mixing | 200 | 15.0 | 584 | 0 | 3 | 24 |
| Example 1 | BA50 | Avicel | Mixing | 200 | 3.0 | 224 | 60 | 6 | 10 |
| Comparative Example 5 | BA50 | Avicel | Mixing | 190 | 25.0 | 559 | 9 | 19 | 31 |
| Example 2 | BA50 | Avicel | Mixing | 190 | 15.0 | 359 | 61 | 4 | 16 |
| Example 3 | BA50 | Avicel | Mixing | 190 | 5.0 | 159 | 64 | 9 | 11 |
| Comparative Example 6 | BA50 | Avicel | Mixing | 190 | 0.0 | 59 | 5 | 3 | 2 |
| Example 4 | BA50 | Avicel | Mixing | 180 | 20.0 | 215 | 69 | 9 | 9 |
| Example 5 | BA50 | Avicel | Mixing | 180 | 10.0 | 115 | 60 | 6 | 12 |
| Comparative Example 7 | BA50 | Avicel | Mixing | 180 | 40.0 | 415 | 18 | 20 | 25 |
| Comparative Example 8 | BA50 | Avicel | Mixing | 180 | 0.0 | 15 | 2 | 1 | 1 |
| Comparative Example 9 | BA50 | Avicel | Mixing | 170 | 30.0 | 0 | 6 | 1 | 1 |
| Comparative Example 10 | BA50 | Avicel | Mixing | 170 | 10.0 | 0 | 4 | 2 | 0 |
| Comparative Example 11 | BA50 | Avicel | Mixing | 170 | 0.0 | 0 | 1 | 0 | 0 |

(continued)

| | Reaction conditions | | | | | | Results | | |
|---|---|---|---|---|---|---|---|---|---|
| | Catalyst | Substrate | Pulverization | Temperature (°C) | Time (min) | Temperature -time product (°C·min) | Yield of G3 to G6 (%) | Yield of G2 (%) | Yield of glucose (%) |
| Comparative Example 12 | None | Avicel | Not performed | 180 | 20.0 | 215 | 5 | 1 | 1 |
| Example 6 | SD50 | Avicel | Mixing | 180 | 20.0 | 215 | 60 | 10 | 11 |
| Example 7 | ZN50 | Avicel | Mixing | 180 | 20.0 | 215 | 60 | 11 | 16 |

In FIG. 3, the results of Examples 1 to 7 and Comparative Examples 1 to 12 carried out at various reaction temperatures for various retention times with the steam-activated carbons (BA50 and SD50) and the chemical-activated carbon (Zn60) are collectively shown. In the figure, the mark "•" means that the yield of the oligomers each having a degree of polymerization of glucose of from 3 to 6 is 50% or more, and the mark "×" means that the yield of the oligomers each having a degree of polymerization of glucose of from 3 to 6 is less than 50%.

The yield of the oligosaccharides each having a degree of polymerization of glucose of 3 to 6 in the reaction performed in the reaction temperature region of from 170°C to 230°C using the pulverized raw material having mixed therein the steam-activated carbon BA50 was: 60% under the conditions of a reaction temperature of 200°C and a reaction time of 3 minutes (Example 1); 61% under the conditions of 190°C and 15 minutes (Example 2); 64% under the conditions of 190°C and 5 minutes (Example 3); and 69% under the conditions of 180°C and 20 minutes (Example 4). Thus, it was confirmed that a high yield of 60% or more was obtained by taking an appropriate reaction time at about 200°C or less. The yield in the reaction performed by taking a reaction time at a high-temperature condition of more than 200°C was: 0% under the conditions of a reaction temperature of 230°C and a reaction time of 0 minutes (Comparative Example 1); 16% under the conditions of 220°C and 0 minutes (Comparative Example 2); and 0% under the conditions of 210°C and 10 minutes (Comparative Example 3). In each of the comparative examples, excessive degradation proceeded, and a high yield of the oligosaccharides each having a degree of polymerization of glucose of from 3 to 6 was not obtained.

In addition, even under the condition of 200°C or less, the yield was: 0% under the conditions of 200°C and 15 minutes (Comparative Example 4); 9% under the conditions of 190°C and 25 minutes (Comparative Example 5); and 18% under the conditions of 180°C and 40 minutes (Comparative Example 7). Due to the excessively long time taken, excessive degradation proceeded, and a high yield was not obtained. Conversely, the yield was: 5% under the conditions of 190°C and 0 minutes (Comparative Example 6); 2% under the conditions of 180°C and 0 minutes (Comparative Example 8); 6% under the conditions of 170°C and 30 minutes (Comparative Example 9); 4% under the conditions of 170°C and 10 minutes (Comparative Example 10); and 1% under the conditions of 170°C and 0 minutes (Comparative Example 11). In these comparative examples, a high yield was not obtained presumably because the hydrolysis did not proceed owing to an insufficient reaction time.

When the reaction was performed at a reaction temperature of 180°C for a reaction time of 20 minutes using activated carbon other than BA50, i.e., SD50 or ZN60, the steam-activated carbon SD50 (Example 6) provided a yield of the oligosaccharides each having a degree of polymerization of glucose of from 3 to 6 of 60%, and the chemical-activated carbon ZN60 (Example 7) also provided a result of 60%. Thus, it was confirmed that each of the steam-activated carbon and the chemical-activated carbon provided a high yield.

The effects of the presence and absence of a catalyst in the reaction at a reaction temperature of 180°C for a reaction time of 20 minutes are compared below. The yield of the oligosaccharides each having a degree of polymerization of glucose of from 3 to 6 was 69% in Example 4 carried out with the raw material simultaneously pulverized with BA50, whereas the yield was 5% in Comparative Example 12 using only Avicel in the absence of the catalyst. It is found from the results that a carbon catalyst is needed in order to obtain the oligosaccharides each having a degree of polymerization of glucose of from 3 to 6 in high yield.

Industrial Applicability

According to the method of the present invention, the cellooligosaccharide having a degree of polymerization of glucose of from 3 to 6, which has heretofore not been industrially produced by the hydrolysis method for plant biomass using a carbon catalyst, can be manufactured, and an additive having a higher function can be provided in the fields of medicines, cosmetics, food, and feed.

**Claims**

1. A method of manufacturing a cellooligosaccharide containing an oligomer having a degree of polymerization of from 3 to 6, the method comprising performing a reaction involving heating and hydrolyzing plant biomass in coexistence with a carbon catalyst and water under such a condition that a temperature-time product over a range of from 170°C to 230°C in a graph for showing a relationship between a reaction temperature on an axis of ordinate and a reaction time on an axis of abscissa is from 80°C·min to 365°C·min.

2. The method of manufacturing a cellooligosaccharide according to claim 1, in which a maximum reaction temperature (X) and a retention time (Y) at the maximum reaction temperature satisfy a relationship represented by the following expressions (1) to (3):

$$Y > -0.0714X + 15 \qquad (1)$$

$$Y < -X + 210 \qquad (2)$$

$$X > 175 \qquad (3)$$

where X represents the maximum reaction temperature (°C), and Y represents the retention time (min) at the maximum reaction temperature.

3. The method of manufacturing a cellooligosaccharide according to claim 1 or 2, in which a temperature increase rate during heating to the maximum reaction temperature, and a cooling rate during cooling from the maximum reaction temperature are each from 0.1°C/min to 30°C/min.

4. The method of manufacturing a cellooligosaccharide according to any one of claims 1 to 3, in which the carbon catalyst is activated carbon.

5. The method of manufacturing a cellooligosaccharide according to any one of claims 1 to 4, in which the carbon catalyst is steam-activated carbon.

6. The method of manufacturing a cellooligosaccharide according to any one of claims 1 to 4, in which the carbon catalyst is air-oxidized steam-activated carbon and/or air-oxidized chemical-activated carbon.

7. The method of manufacturing a cellooligosaccharide according to any one of claims 1 to 6, in which the plant biomass and the carbon catalyst are mixed and pulverized in advance before being used as a raw material.

8. The method of manufacturing a cellooligosaccharide according to any one of claims 1 to 7, in which the plant biomass is pulverized in advance before being used as a raw material.

9. The method of manufacturing a cellooligosaccharide according to any one of claims 1 to 8, in which the reaction is terminated at a time point when a yield of an oligosaccharide having a degree of polymerization of glucose of from 3 to 6 is from 50% to 80%.

FIG. 1

FIG. 2

FIG. 3

Reaction Temperature (°C)

## INTERNATIONAL SEARCH REPORT

| International application No. |
| --- |
| PCT/JP2016/087161 |

**A. CLASSIFICATION OF SUBJECT MATTER**
*C07H3/06*(2006.01)i, *B09B3/00*(2006.01)i, *C08B15/02*(2006.01)i, *C13B10/00*(2011.01)i, *C13K13/00*(2006.01)i, *C07B61/00*(2006.01)n

According to International Patent Classification (IPC) or to both national classification and IPC

**B. FIELDS SEARCHED**

Minimum documentation searched (classification system followed by classification symbols)
C07H3/06, B09B3/00, C08B15/02, C13B10/00, C13K13/00, C07B61/00

Documentation searched other than minimum documentation to the extent that such documents are included in the fields searched
Jitsuyo Shinan Koho          1922–1996   Jitsuyo Shinan Toroku Koho   1996–2017
Kokai Jitsuyo Shinan Koho    1971–2017   Toroku Jitsuyo Shinan Koho   1994–2017

Electronic data base consulted during the international search (name of data base and, where practicable, search terms used)
JSTPlus/JMEDPlus/JST7580(JDreamIII), CAplus/REGISTRY(STN)

**C. DOCUMENTS CONSIDERED TO BE RELEVANT**

| Category* | Citation of document, with indication, where appropriate, of the relevant passages | Relevant to claim No. |
| --- | --- | --- |
| X/Y | JP 2006-129735 A (Hiroshima University), 25 May 2006 (25.05.2006), claims; examples (Family: none) | 1-9/1-9 |
| X/Y | WO 2014/097800 A1 (Showa Denko Kabushiki Kaisha), 26 June 2014 (26.06.2014), claims; paragraphs [0013] to [0020], [0025] to [0029]; examples & US 2015/0337402 A1 claims; paragraphs [0023] to [0037], [0046] to [0052]; examples | 1-8/1-9 |

☒ Further documents are listed in the continuation of Box C.      ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 01 March 2017 (01.03.17) | 14 March 2017 (14.03.17) |

| Name and mailing address of the ISA/ Japan Patent Office 3-4-3, Kasumigaseki, Chiyoda-ku, Tokyo 100-8915, Japan | Authorized officer |
| --- | --- |
| | Telephone No. |

Form PCT/ISA/210 (second sheet) (January 2015)

**INTERNATIONAL SEARCH REPORT**

International application No.

PCT/JP2016/087161

C (Continuation). DOCUMENTS CONSIDERED TO BE RELEVANT

| Category* | Citation of document, with indication, where appropriate, of the relevant passages | Relevant to claim No. |
|---|---|---|
| X/Y | WO 2012/128055 A1 (Showa Denko Kabushiki Kaisha), 27 September 2012 (27.09.2012), paragraphs [0016] to [0024], [0029] to [0032]; examples & US 2014/0007862 A1 paragraphs [0035] to [0045], [0050] to [0054]; examples & EP 2689862 A1 & CN 103442816 A | 1-8/1-9 |
| X/Y | WO 2011/036955 A1 (National University Corporation Hokkaido University), 31 March 2011 (31.03.2011), claims; paragraphs [0047] to [0058]; examples & EP 2481479 A1 claims; paragraphs [0047] to [0058]; Embodiments & US 9144785 B2 & CN 102711994 A | 1-8/1-9 |
| Y | JP 2009-57354 A (National Institute of Advanced Industrial Science and Technology), 19 March 2009 (19.03.2009), claims; paragraph [0046]; examples 4, 6, 7 (Family: none) | 1-9 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009189293 A
- JP 2011068578 A
- US 2012232264 A1
- WO 2011036955 A1
- US 9144785 B2
- WO 2012128055 A1
- US 9284614 B2
- JP 2006320261 A